# FASCICULE DE BREVET EUROPEEN

(11) **EP 1 148 865 B1**
(45) Date de publication et mention de la délivrance du brevet: **25.09.2002**
(21) Numéro de dépôt: 00901686.6
(22) Date de dépôt: 31.01.2000
(51) Int. Cl.: A61K 7/155

(54) **REVETEMENT POUR MATIERES THERMOPLASTIQUES POUR EPILATION**
BESCHICHTUNG FUR THERMOPLASTEN ZUR ENTHAARUNG
COATING FOR THERMOPLASTIC DEPILATORY MATERIAL

(30) Priorité: 02.02.1999 FR 9901462
(43) Date de publication de la demande: 31.10.2001
(73) Titulaire: SEB S.A., 69130 Ecully (FR)
(72) Inventeur: CATHAUD, Muriel, F-69780 Vénissieux (FR); MOREAU, Pierre, F-87000 Limoges (FR)
(74) Mandataire: Kiehl, Hubert
(86) Numéro de dépôt international: FR0000222
(87) Numéro de publication internationale: WO00045783

(56) Documents cités:
- EP-A- 0 194 181
- EP-A- 0 693 279
- FR-A- 2 776 924

## Description

La présente invention est relative au domaine technique des matières thermoplastiques, notamment des cires utilisées pour l'épilation. Cette catégorie de cires est utilisée dans des distributeurs de cire tels que par exemple des applicateurs de cire à rouleau.

De telles cires sont déposées en couche mince sur la peau à l'aide d'un distributeur après avoir été fondues ou ramollies. La couche de cire une fois solidifiée emprisonne les poils et peut être retirée. Certaines cires, telles que celles connues du document EP 0 299 816, présentent l'avantage d'être autoporteuses. Avec de telles cires, la couche de cire une fois solidifiée peut être retirée sans utiliser de support tel qu'une bande de tissus ou de cellophane.

Un distributeur de cire à épiler du type applicateur à rouleau est connu du document EP 0 629 366 au nom de la demanderesse. Cet appareil comporte un réservoir associé à des moyens de chauffe et un rouleau prévu pour distribuer la cire fondue en couche mince sur la peau. La cire utilisée dans de tels distributeurs se présente généralement sous la forme de grains ou granules, par exemple des grains de forme sensiblement ellipsoïdale aplatie dont le diamètre est de l'ordre de 4 à 5 mm et la hauteur de 2 à 3 mm, de pastilles de quelques millimètres cubes à quelques centimètres cubes ou encore de pains.

Les cires, notamment celles dites basse température, présentent, dans certaines conditions d'usage ou de stockage, des inconvénients liés à la fusion des couches de surface.

On connaît, de la demande de brevet FR 9804509 (FR-A-2 776 924) au nom de la demanderesse, un assemblage de matières fusibles pour épilation où les granules de cire sont constitués par une matière formant noyau comportant une couche d'enrobage en une matière dont le point de ramollissement est plus élevé que celui du noyau. L'enrobage donnant entièrement satisfaction pour sa tenue en température, on s'est toutefois aperçu qu'il y avait des problèmes d'accrochage entre les granules de cire. L'accrochage empêche le glissement des granules l'un sur l'autre gênant ainsi leur écoulement donc la facilité de remplissage du réservoir de l'appareil à épiler ou du sachet de conditionnement de la cire.

Il est connu des produits médicamenteux qui utilisent des revêtements facilitant leur conditionnement et permettant de maintenir leur forme à la température ambiante. Cependant, le problème de la tenue en température ne se pose pas pour ces produits qui doivent normalement être stockés à une température proche, voire inférieure à la température ambiante.

Les cires à épiler présentent, dans certaines conditions d'usage ou de stockage, des inconvénients liés au ramollissement des couches de surface. La température des mains est suffisante pour provoquer le phénomène. La surface de la cire devient poisseuse et/ou graisseuse, ce qui rend la manipulation peu agréable. Ces conditions peuvent être également rencontrées lorsque les températures de stockage préconisées ne sont pas respectées, ce qui arrive surtout en été, période pendant laquelle on s'épile le plus. Les grains de cire peuvent alors s'agglomérer, ce qui rend leur utilisation ultérieure moins aisée. Les grains, mais aussi les pains de cire, peuvent voir leur forme se modifier sous l'action du ramollissement des couches externes de la cire, engendrant ainsi une gêne à l'utilisation qui peut être prévue dans un espace spécialement défini.

Des problèmes de maintien en température de la forme des granules ou grains de cire à épiler sont également rencontrés lors de la manipulation de la cire en vue du remplissage du réservoir d'un distributeur. La température dégagée par les parois chaudes du réservoir lors du remplissage peut entraîner le ramollissement de la surface des grains, ce qui provoque une agglomération de ces derniers à l'entrée du réservoir et rend le remplissage difficile et cela de façon d'autant plus importante que les grains resteront plus longtemps dans l'entrée car ils s'écoulent moins vite.

La présente invention vise à fournir des matières thermoplastiques pour épilation remédiant aux inconvénients précités.

Le but de l'invention est d'obtenir des matières thermoplastiques sous forme de granules de cire qui ne collent pas, qui peuvent glisser facilement l'un par rapport à l'autre et/ou par rapport à une paroi fixe et qui présentent une bonne tenue en température.

Un autre but de l'invention est d'obtenir de telles propriétés pour la matière thermoplastique d'une manière simple et peu coûteuse.

Un but supplémentaire de l'invention est d'obtenir des granules de matière thermoplastique comportant des composants de coloration, aromatiques ou des composants actifs à propriétés anti-irritantes, anti-rougeur, antiseptiques, adoucissantes ou ayant d'autres propriétés bénéfiques pour la peau.

Les objets assignés à l'invention sont atteints par un revêtement pour matières thermoplastiques pour épilation, caractérisé en ce qu'il consiste en une couche de matériau pulvérulent à propriétés lubrifiantes recouvrant un noyau de cire à épiler.

Ainsi les grains, pastilles ou granules de matière thermoplastique comportant ce revêtement, présentent une tenue en température améliorée. Le matériau pulvérulent forme une couche agissant comme une barrière qui sépare les grains de cire et empêche leur collage. De plus, le matériau pulvérulent étant constitué de particules très fines n'adhérant pas entre elles, ces particules forment un support de roulement des grains de cire qui glissent l'un par rapport à l'autre, ce qui confère une manipulation aisée des grains de cire.

Avantageusement, le matériau de revêtement est du talc.

Ce matériau présente l'avantage d'être finement divisé et ainsi de bien couvrir les grains, d'être facile à utiliser, de pouvoir être utilisé pour des soins d'hygiène et cosmétiques, et de présenter une sensation de sec, de lisse et de douceur au toucher. De surcroît, on a remarqué que, lors de la fusion de la cire, il agit comme un épaississant, augmentant la viscosité de cette dernière facilitant ainsi la dépose sur la peau.

De préférence, le noyau dudit revêtement se présente sous forme de grains, billes ou pastilles dont le diamètre est supérieur à 1 mm, les grains ayant une forme sphérique ou angulaire.

Ceci facilite le remplissage du réservoir de l'appareil, le conditionnement en sachets ou boîtes, permettant aux grains de bien remplir un volume donné.

Avantageusement, les grains de cire sont recouverts de quelques dixièmes de pourcentage à quelques pourcentages en poids de matériau pulvérulent.

Ainsi, on obtient une couche de faibles dimensions, mais avec de bonnes propriétés de tenue en température. On remarque aussi qu'une quantité réduite de matériau pulvérulent suffit, ce qui réduit les coûts de fabrication d'un tel revêtement.

Selon une variante avantageuse de réalisation de l'invention, la couche du revêtement pour matières thermoplastiques pour épilation comprend un colorant.

Ainsi, on peut largement diversifier la présentation de la cire, lui donner un aspect agréable et cosmétique, ou associer à une couleur des propriétés spécifiques de sa composition.

De préférence, ledit colorant est le mica et/ou l'oxyde de titane. Ceci confère une coloration agréable, blanche nacrée, en utilisant des produits qui ne sont pas agressifs pour la peau.

Avantageusement, la couche de revêtement contient des produits cosmétiques ou pharmaceutiques encapsulés.

Ces produits deviennent actifs ou sont libérés seulement lorsqu'ils sont portés à une certaine température qui sera atteinte juste avant la dépose sur la peau et, comme ils ne sont pas dispersés ou dilués dans la masse de cire, mais qu'ils appartiennent au revêtement, ils restent concentrés par endroit et ils ont une action plus prononcée, rapide et bénéfique sur la peau, en étant tout de même utilisés en petite quantité.

Les produits actifs sont le plus souvent sensibles à la température. Lorsqu'ils dépassent un seuil limite de température (inférieur à 100°C), ils se dégradent ou ils perdent une partie de leur efficacité, ils sont donc thermosensibles. Les matières dans lesquelles ils doivent avantageusement être introduits sont pour leur part fabriquées à des températures élevées (supérieures à 100°C). L'introduction des produits actifs en cours de préparation reviendrait à les dégrader par la température.

Avantageusement les produits actifs seront donc introduits au produit à la fin de leur fabrication et seront maintenus à la surface des granules.

Selon une autre variante avantageuse, la couche du revêtement selon l'invention comprend un produit aromatique.

Un tel produit dégage une odeur agréable pendant l'épilation, ce qui parfume la peau et qui rend l'épilation plus plaisante.

Selon encore une autre variante de l'invention, la couche de revêtement comprend un produit anti-irritation et/ou un produit anti-rougeur et/ou un produit antiseptique.

Ainsi, ce produit présent dans la couche de revêtement vient en contact avec la peau la protégeant ainsi contre les irritations ou les rougeurs provoquées par la cire à épiler ou il a une action cicatrisante.

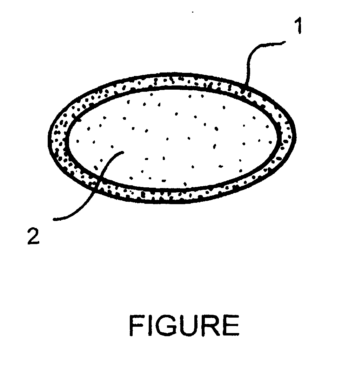

L'invention sera mieux comprise à l'étude de modes de réalisation pris à titre nullement limitatifs et illustrés dans la figure annexée qui représente une vue schématique en coupe longitudinale d'une pastille ou d'un grain de matériau thermoplastique comportant un revêtement selon l'invention.

Le revêtement pour matières thermoplastiques pour épilation selon l'invention comporte une couche 1 de matériau pulvérulent à propriétés lubrifiantes recouvrant un noyau 2 composé de cire à épiler.

Le matériau pulvérulent utilisé pour enrober le noyau 2 de cire à épiler est de préférence du talc.

Le talc est une poudre très fine de silicate naturel de magnésium, de couleur blanche-grisâtre, sans odeur, cristalline. Les particules de talc étant très fines, le diamètre pouvant varier de quelques microns à quelques dixièmes de micron, il a la propriété de bien envelopper une surface et de créer une barrière mécanique entre deux surfaces. Comme on ne peut pratiquement pas parler d'un point de fusion du talc, la barrière qu'il forme entre les surfaces, notamment celles des grains de cire, confère à ces derniers une bonne tenue en température. Ceci devient impératif quand la cire n'est pas stockée dans des conditions optimales, notamment quand la température ambiante devient supérieure à 30°C.

De surcroît, de par sa constitution pulvérulente, il agit comme un très bon lubrifiant facilitant non seulement l'éloignement des grains les uns par rapport aux autres, mais aussi un très bon glissement de ces derniers. En conclusion, les grains glissent sans s'accrocher et ils maintiennent cette propriété en température.

Le talc peut être déposé sur les grains, granules, pastilles ou pains de cire par saupoudrage, par pulvérisation ou tout simplement en le mélangeant avec les grains de cire. Ceci confère une mise en oeuvre facilitée, surtout pour la production en grande série des matériaux thermoplastiques comportant un tel revêtement.

L'épaisseur d'une couche de matériau pulvérulent déposé par un des procédés mentionnés fait que les grains de cire 2 soient recouverts de quelques dixièmes de pourcentage à quelques pourcentages en poids de matériau pulvérulent 1.

D'autres matériaux pulvérulents pourraient être utilisés, tels le mica, l'oxyde de titane, le mica recouvert d'oxyde de titane, le kaolin. Ces produits peuvent être mélangés au talc, ou entre eux ou utilisés en tant que tels pour recouvrir les grains de cire.

Le noyau 2 de cire à épiler se présente sous forme de grains, billes, pastilles ou pains dont le diamètre équivalent est de préférence supérieur à 1 mm. II peut présenter des formes arrondies, par exemple sphérique, ellipsoïdale, ou également des formes angulaires.

Des exemples de matières sont donnés ci-après pour la cire à épiler du noyau 2.

Selon un premier exemple de réalisation, le noyau 2 est formé par une cire à épiler classique, d'origine végétale ou synthétique, ou à base de résine naturelle ou synthétique, présentant une plage de ramollissement de 70 à 80°C.

Selon un deuxième exemple de réalisation, le noyau 2 est formé par une cire à base de sucre présentant une plage de ramollissement de 50 à 60°C.

A titre de variante d'autres matières fusibles présentant une résistance mécanique suffisante pour former une couche autoporteuse peuvent être utilisées pour le noyau 2.

Afin d'augmenter les propriétés de glissement des grains de cire, on peut recouvrir de matériau pulvérulent lubrifiant des grains de cire présentant déjà une couche d'enrobage lui conférant des propriétés de tenue en température, tel que décrit dans la demande de brevet FR 9804509 au nom de la demanderesse.

Le matériau d'enrobage, par exemple le talc peut être mélangé avec tout colorant pulvérulent possible afin de changer l'aspect extérieur de la cire. Par exemple, un mélange de talc, du mica et de l'oxyde de titane confère à la cire un aspect perlé, nacré très agréable.

Selon une variante avantageuse de l'invention, le matériau pulvérulent lubrifiant peut être mélangé avec des produits cosmétiques ou pharmaceutiques encapsulés. Ces produits peuvent être des produits aromatiques encapsulés qui dégagent une odeur agréable lorsqu'ils sont portés en température, par exemple essence de lavande, ou une autre essence de fleurs, fruits, etc.

On peut mélanger au matériau pulvérulent de base, notamment le talc, des produits anti-irritaton et/ou anti-rougeur et/ou antiseptiques et/ou adoucissants également encapsulés. Ces produits agissent en tant que produits actifs lors de l'épilation, car ils se trouvent concentrés à l'extérieur de la matière, non pas dispersés ou dilués dans la masse de cire. De tels produits constituent un bénéfice supplémentaire à la cire lors de l'épilation qui, de surcroît, ont gardé l'ensemble de leur propriétés non dégradées par la chaleur lors de la fabrication de la cire. Dans cet agencement, il est bien entendu que les quantités utilisées, pour amener ces produits en contact avec la peau, sont beaucoup plus faibles que si l'on noyait les mêmes produits dans la masse de cire où ils seraient très dilués.

A titre d'exemple ces actifs peuvent être des apaisants et anti-inflammatoires comme par exemple I' " Alpha-bisabilol " ou I' " Amanduline " des Laboratoires SILAB ou le " Biophytex " des Laboratoires Sérobiologiques.

Des actifs anti-repousse du poil, tels que par exemple le " Pilinhib" des Laboratoires Sérobiologiques peuvent également être ajoutés.

## Revendications

1. Revêtement pour matières thermoplastiques pour épilation, **caractérisé en ce qu'**il consiste en une couche (1) de matériau pulvérulent à propriétés lubrifiantes recouvrant un noyau (2) de cire à épiler.

2. Revêtement pour matières thermoplastiques pour épilation selon la revendication 1, **caractérisé en ce que** le matériau de revêtement (1) est un talc.

3. Revêtement pour matières thermoplastiques pour épilation selon l'une des revendications précédentes , **caractérisé en ce que** le noyau (2) se présente sous forme de grains, billes, pastilles ou pains dont le diamètre équivalent est supérieur à 1 mm.

4. Revêtement pour matières thermoplastiques pour épilation selon la revendication 3, **caractérisé en ce que** le noyau (2) est de forme sphérique ou angulaire.

5. Revêtement pour matières thermoplastiques pour épilation selon l'une des revendications précédentes, **caractérisé en ce que** les grains de cire (2) sont recouverts de quelques dixièmes de pourcentage à quelques pourcentages en poids de matériau pulvérulent (1).

6. Revêtement pour matières thermoplastiques pour épilation selon l'une des revendications précédentes, **caractérisé en ce que** la couche (1) comprend un colorant.

7. Revêtement pour matières thermoplastiques pour épilation selon la revendication 6, **caractérisé en ce que** ledit colorant est le mica et/ou l'oxyde de titane.

8. Revêtement pour matières thermoplastiques pour épilation selon la revendication 1, **caractérisé en ce que** la couche (1) contient des produits cosmétiques ou pharmaceutiques encapsulés.

9. Revêtement pour matières thermoplastiques pour épilation selon la revendication 8, **caractérisé en ce que** la couche (1) contient un produit aromatique.

10. Revêtement pour matières thermoplastiques pour épilation selon la revendication 8, **caractérisé en ce que** la couche (1) contient des produits de type anti-irritation et/ou anti-rougeur et/ou antiseptiques et/ou adoucissants.

## Patentansprüche

1. Beschichtung für Thermoplaste zu Epilierzwecken, **dadurch gekennzeichnet, daß** sie aus einer Schicht (1) eines gleitfähigen pulverförmigen Materials besteht, die einen Kern (2) aus Epilierwachs überdeckt.

2. Beschichtung für Thermoplaste zu Epilierzwecken nach Anspruch 1, **dadurch gekennzeichnet, daß** das Beschichtungsmaterial (1) ein Talkum ist.

3. Beschichtung für Thermoplaste zu Epilierzwecken nach einem der vorstehenden Ansprüche, **dadurch gekennzeichnet, daß** der Kern (2) die Form von Körnern, Kugeln, Tabletten- oder Stücken aufweist, deren äquivalenter Durchmesser größer ist als 1 mm.

4. Beschichtung für Thermoplaste zu Epilierzwecken nach Anspruch 3, **dadurch gekennzeichnet, daß** der Kern (2) eine sphärische oder eckige Form aufweist.

5. Beschichtung für Thermoplaste zu Epilierzwecken nach einem der vorstehenden Ansprüche, **dadurch gekennzeichnet, daß** die Wachskörner (2) mit einigen Zehntel bis einigen Gewichtsprozenten pulverförmigen Materials (1) beschichtet sind.

6. Beschichtung für Thermoplaste zu Epilierzwecken nach einem der vorstehenden Ansprüche, **dadurch gekennzeichnet, daß** die Schicht (1) einen Farbstoff enthält.

7. Beschichtung für Thermoplaste zu Epilierzwecken nach Anspruch 6, **dadurch gekennzeichnet, daß** der Farbstoff Glimmer und/oder Titanoxid ist.

8. Beschichtung für Thermoplaste zu Epilierzwecken nach Anspruch 1, **dadurch gekennzeichnet, daß** die Schicht (1) eingekapselte kosmetische oder pharmazeutische Produkte enthält.

9. Beschichtung für Thermoplaste zu Epilierzwecken nach Anspruch 8, **dadurch gekennzeichnet, daß** die Schicht (1) einen Aromastoff enthält.

10. Beschichtung für Thermoplaste zu Epilierzwecken nach Anspruch 8, **dadurch gekennzeichnet, daß** die Schicht (1) Stoffe einer reizungs- und/oder rötungshemmenden und/oder antiseptischen und/oder weichmachenden Art enthält.

## Claims

1. A coating for thermoplastic depilatory materials, the coating being **characterized in that** it consists in a layer (1) of powder material having lubricating properties covering a core (2) of depilatory wax.

2. A coating for thermoplastic depilatory materials according to claim 1, **characterized in that** the coating material (1) is talc.

3. A coating for thermoplastic depilatory materials according to either preceding claim, **characterized in that** the core (2) is in the form of grains, beads, pellets, or tablets having an equivalent diameter greater than 1 mm.

4. A coating for thermoplastic depilatory materials according to claim 3, **characterized in that** the core (2) is spherical or angular in shape.

5. A coating for thermoplastic depilatory materials according to any preceding claim, **characterized in that** the grains of wax (2) are covered in several tenths of a percent to several percent by weight of powder material (1).

6. A coating for thermoplastic depilatory materials according to any preceding claim, **characterized in that** the layer (1) includes a coloring agent.

7. A coating for thermoplastic depilatory materials according to claim 6, **characterized in that** said coloring agent is mica and/or titanium oxide.

8. A coating for thermoplastic depilatory materials according to claim 1, **characterized in that** the layer (1) contains encapsulated cosmetic or pharmaceutical substances.

9. A coating for thermoplastic depilatory materials according to claim 8, **characterized in that** the layer (1) contains an aromatic substance.

10. A coating for thermoplastic depilatory materials according to claim 8, **characterized in that** the layer (1) contains substances of the anti-irritant and/or anti-reddening and/or antiseptic and/or softening type.
